(19) Europäisches Patentamt
European Patent Office
Office européen des brevets

(11) Veröffentlichungsnummer: **0 091 114**
**B1**

# (12) EUROPÄISCHE PATENTSCHRIFT

(45) Veröffentlichungstag der Patentschrift: **04.06.86**

(51) Int. Cl.⁴: **C 07 C 51/41**, C 07 F 7/30, C 07 C 101/22, C 07 D 307/62

(21) Anmeldenummer: **83103285.9**

(22) Anmeldetag: **02.04.83**

## (54) Pharmazeutisch wirksame organische Germaniumverbindungen.

(30) Priorität: **06.04.82 DE 3212817**

(43) Veröffentlichungstag der Anmeldung: **12.10.83 Patentblatt 83/41**

(45) Bekanntmachung des Hinweises auf die Patenterteilung: **04.06.86 Patentblatt 86/23**

(84) Benannte Vertragsstaaten: **AT BE CH FR GB IT LI LU NL SE**

(73) Patentinhaber: **SANUM-KEHLBECK GmbH & Co. KG, Bahnhofstrasse 2, D-2812 Hoya (DE)**

(72) Erfinder: **Kehlbeck, Heinrich, Bahnhofstrasse 2, D-2812 Hoya (DE)**
Erfinder: **Lekim, Dac, Dr., Esch, Ringstrasse 33, D-5000 Köln 71 (DE)**

(74) Vertreter: **Rücker, Wolfgang, Dipl.-Chem., Hubertusstrasse 2, D-3000 Hannover 1 (DE)**

(56) Entgegenhaltungen:
**FR - A - 1 325 217**

**JOURNAL OF CHEMICAL SOCIETY, J.C.S. DALTON, 1976, Seiten 2238-42, N.W. ALCOCK et al.: "Acetates and acetato-complexes. Part 1. Preparation of acetatocomplexes and conductimetric studies in the acetic anhydride solvent system"**
**CHEMICAL ABSTRACTS, Band 73, Nr. 1, 6. Juli 1970, Seite 291, Nr. 3430h, Columbus, Ohio, USA, L.S. MEL'NICHENKO et al.: "Synthesis of tin and germanium tetraacylates"**
**CHEMICAL ABSTRACTS, Band 67, Nr. 24, 11. Dezember 1967, Seite 10561, Nr.111850e, Columbus, Ohio, USA,E.M. BELOUSOVA et al.: "Composition and ionization constants of tartrato-germanic acid and citratogermanic acid"**

(56) Entgegenhaltungen: (Fortsetzung)
**CHEMICAL ABSTRACTS, Band 83, Nr. 8, 25. August 1975, Seite 515, Nr. 66359u, Columbus, Ohio, USA, A.F. POZHARITSKII et al.: "Composition and some properties of complexes of germanium (IV) with glycolic and lactic acids"**
**CHEMICAL ABSTRACTS, Band 81, Nr. 1, 8. Juli 1974, Seite 345, Nr. 4172c, Columbus, Ohio, USA, A.F. POZHARITSKII et al.: "Composition and structure of tartratogermanic acid"**

**EP 0 091 114 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf physiologisch wirksame organische Germaniumkomplexe und Verfahren zu ihrer Herstellung.

Germanium wird vorwiegend in der Elektronikindustrie eingesetzt, wo das Metall in höchster Reinheit verlangt wird. Die biologischen Eigenschaften von Germanium und dessen Verbindungen sind bislang weitgehend unbekannt. Germaniumoxid wurde z.B. als blutbildendes Mittel bei Anämie versuchsweise eingesetzt (s. Hagers Handbuch der pharmazeutischen Praxis, 4. Auflage 1972–1980, Springer Verlag).

In der japanischen Patentschrift 79 136 220 (s. Chemical Abstracts, Vol. 92, 1980, Ref. Nr. 92: 135 430 k) beschreibt Toru Hamada eine Salbe mit 99,99% reinem Germanium in metallischer Form, mit antiinflammatorischer Wirkung. Die deutsche Patentschrift 2 634 900 hat zum Gegenstand 3-Trihydroxygermanylpropionsäure, ihre Salze und das Verfahren zu deren Herstellung. In dieser Verbindung tritt Germanium direkt mit Kohlenstoff in eine relativ stabile Germanium-Kohlenstoffbindung:

$$\mathrm{HO{-}\overset{\displaystyle OH}{\underset{\displaystyle OH}{Ge}}{-}CH_2{-}CH_2{-}COO\ R}$$

R = Li oder H

Diese Verbindungsreihe soll ein sehr breites pharmakologisches Wirksamkeitsspektrum besitzen (positive Wirkung gegen physische und neurologische Störungen, Stoffwechselstörungen, Hautkrankheiten, Nierenfunktionsstörung usw.).

In den Veröffentlichungen

Zhurnal neorganiceskoj khimij, Vol. 12 (1967) S. 1846–1850;

Zhurnal obshchej khimij, Vol. 44 (1974) S. 549–553;

Zhurnal obshchej khimij, Vol. 45 (1975) S. 1038–1043,

sind Komplexe beschrieben aus Germanium(IV)-Oxid oder -hydroxid mit Weinsäure oder Zitronensäure bzw. Glycolsäure oder Milchsäure. Eine pharmakologische Wirkung dieser Komplexe wird nicht beschrieben.

Aufgabe der vorliegenden Erfindung ist es, Germanium in Form leicht wasserlöslicher Komplexe mit organischen Carbonsäuren herzustellen, die biologische und pharmakologische Eigenschaften besitzen und solche Komplexe als Arzneimittel zu verwenden.

In Verfolg des Erfindungsgedankens werden solche Komplexe auch als Zytostatika verwendet.

Wir haben nunmehr überraschenderweise festgestellt, dass Germanium auf einfache Art und Weise mit Ascorbinsäure oder wasserlöslichen organischen Carbonsäuren aller Art in Verbindung treten kann. Diese Germaniumkomplexe sind leicht löslich in Wasser, haben kaum toxische Wirkung und besitzen ausgezeichnete biologische und pharmakologische Eigenschaften.

Gegenstand dieser Erfindung sind die Komplexe von Germanium mit Ascorbinsäure oder physiologischen, wasserlöslichen, organischen Carbonsäuren sowie das Verfahren zu deren Herstellung. Als Säuren kommen z.B. in Frage die Aminosäuren wie Asparginsäure, Glutaminsäure usw., ferner die Carbonsäure aus dem Zitronensäurezyklus wie Zitronensäure, Isozitronensäure, Bernsteinsäure, Ketoglutarsäure, Fumarsäure usw., ferner die physiologischen wasserlöslichen Carbonsäuren wie Milchsäure, ferner Ascorbinsäure.

Die akute Toxizität der Germaniumkomplexe mit Zitronensäure und Bernsteinsäure wurde nach der Litchfield-Wilcoxon-Methode bei Mäusen untersucht. Die Substanzen wurden intraperitoneal verabreicht.

Die letale Dosis LD 50/24 Stunden betrug 275 mg/kg Körpergewicht für die Komplexe von Germanium und Bernsteinsäure. Bei Anwendung der gleichen Methode erwies sich Germanium-Zitrat als nicht toxisch (LD 50 grösser als 2 500 mg/kg Körpergewicht).

Die obigen Komplexe wurden dem Allium-Test nach Levan unterworfen. Der Samen von allium œpa L. wurde auf Petrischalen inkubiert. Als die Wurzeln die Länge von 1 cm erreicht hatten, wurden in die Petrischalen wässrige Lösungen der untersuchten Germaniumkomplexe in der Konzentration von 0,0625%, 0,125%, 0,25% und 0,5% gegeben. Das Ergebnis zeigte eindeutig, dass die Germaniumkomplexe eine zytostatische Wirkung besitzen, was mit der Senkung des Mitoseindexes verbunden ist. Diese Wirkung hängt offensichtlich von der Regenerationswirkung auf den Zellkern und von der Mitosestörung ab.

Der Germanium-Asparginsäure-Komplex wurde bei Menschen geprüft. Es wurden 6 Probanden mit der Diagnose von bösartigen Ovarien bzw. Uterus-Tumoren zur stationären Behandlung aufgenommen. Den Patienten wurde 100 mg der Substanz in 10%-iger Lösung zweimal täglich oral verabreicht. Die Tumore wurden operativ entfernt.

Es kann aus den durchgeführten Prüfungen generell geschlossen werden, dass bei allen Patientinnen ein auffallend besseres Wohlbefinden beobachtet wurde. Weiterhin wurde bei 5 der 6 Patientinnen weder Exsudatbildung in der Bauchhöhle noch in der inneren Beckenhöhle festgestellt. Bei einer Patientin wurde nur geringe Exsudatbildung gefunden. Es wurde keine toxische Nebenwirkungen beobachtet. In den post-operativen gynäkologischen Untersuchungen ca. einen Monat nach der Behandlung wurde in allen Patientinnen keine Infiltration verzeichnet.

Eine Mischung mehrerer Germaniumkomplexe mit Milchsäure, Ascorbinsäure und Asparginsäure wurde versuchsweise in der Geriatrie eingesetzt. In vielen Fällen wurde ein verbessertes Wohlgefühl sowie eine schnellere Reaktion gegen akustische und visuelle Stimuli im Vergleich zu der vor der Behandlung beobachtet.

Zusammenfassend kann gesagt werden, dass alle bisher untersuchten Germaniumkomplexe mit physiologischen, wasserlöslichen, organischen Carbonsäuren oder deren Mischungen, eine überraschende, hohe biologische Wirksamkeit – dazu auffallend frei von Nebenwirkungen – besitzen.

Vorzugsweise erfolgt die Herstellung in der Form, dass man direkt von Germaniumoxid ($GeO_2$) ausgeht. Das Germaniumoxid wird mit anorganischen Basen, wie z.B. Natriumhydroxid oder Kaliumhydroxid, erhitzt. Die pulverige Mischung wird abgekühlt, in Wasser suspendiert und mit einer der vorstehend genannten Säuren oder einer Mischung davon versetzt, wobei eine klare Lösung entsteht. Der ph-Wert der Lösung beträgt gewöhnlich 2,0 bis 4,5. Durch Abkühlung, bzw. Zugabe von Alkoholen (Äthanol, Methanol usw.) fällt der entsprechende Germaniumkomplex aus, das Produkt wird abfiltriert und anschliessend mit Alkohol, bzw. Aceton oder Äther gewaschen und bis zum konstanten Gewicht bei Raumtemperatur i. vac. getrocknet.

Die klare Lösung kann auch direkt auf einen bestimmten Germaniumgehalt mit Wasser gebracht werden. Die so standardisierte Lösung – mit oder ohne Zusatz von pharmazeutischen Hilfsstoffen, Geschmackskorrigentien usw. – kann auch sofort sterilfiltriert und in Flaschen, bzw. Ampullen, abgefüllt werden. Die gebrauchsfertige Lösung kann mehrere Gramm Germanium / 100 ml enthalten.

Als geeignete Säure kann Ascorbinsäure oder jede beliebige wasserlösliche, physiologisch unbedenkliche, organische Carbonsäure verwendet werden, vorzugsweise die Aminosäuren wie z.B. Asparginsäure, Glutaminsäure usw. Ketoglutarsäure, Bernsteinsäure usw., ferner die anderen wasserlöslichen physiologischen Carbonsäuren wie Essigsäure, Milchsäure, Pantothensäure usw.

Im folgenden sei die Erfindung an Hand einiger Beispiele näher erläutert.

Beispiel 1

20,8 g Germanium-IV-Oxid wurde mit einer konzentrierten wässrigen Lösung von 22,4 g Kaliumhydroxid vermischt und bis zum Trocknen in einer Porzellanschale erhitzt. Die Mischung wurde abgekühlt und portionsweise mit insg. 600 ml bidest. Wasser in einen Becher überführt.

Die Suspension wurde anschliessend erwärmt und unter Rühren mit ca. 100 g Bernsteinsäure bis zur vollständigen Lösung versetzt. Die Lösung wurde von evtl. partikulärer Verunreinigung warm abfiltriert und langsam abgekühlt. Der Germanium-Bernsteinsäure-Komplex kristallisiert aus und wird abfiltriert. Zur Vervollständigung der Kristallisation wurde das Filtrat abgekühlt und mit einem Volumen Äthanol versetzt.

Ausbeute: 80 g mit einem Germaniumgehalt von 9%, berechnet auf wasserfreier Basis.

Beispiel 2

Analog zum Beispiel 1 wurde eine Lösung mit 62,4 g Germanium-IV-Oxid und ca. 350 g einer äquimolaren Mischung von Ascorbinsäure, Milchsäure und Zitronensäure eine Lösung hergestellt. Die Lösung wurde auf 5 mg Ge/ml standardisiert (insg. 7,2 l) und hat einen ph-Wert von etwa 4,0. Sie wurde sterilfiltriert (0,2 Micron Filter) und in Flaschen à 10 ml unter aseptischen Bedingungen abgefüllt.

Beispiel 3

208 g Germanium-IV-Oxid wurde – wie in Beispiel 1 angegeben – mit einer konzentrierten Lösung von 242 g Kaliumhydroxid in Wasser vermischt. Die Suspension wurde dann bis zur vollständigen Trocknung erhitzt. Die Mischung wurde abgekühlt, in 14 l bidest. Wasser suspendiert und mit 266 g Asparginsäure versetzt. Durch Zugabe von N/10 Salzsäure wurde vollständige Lösung erzielt.

Die Lösung wurde dann von partikulärer Verunreinigung durch Filtration befreit, in Flaschen abgefüllt und sterilisiert. Der Gehalt von Germanium betrug 10 mg/ml.

Beispiel 4

10,4 g Germanium-IV-Oxid wurden – wie in Beispiel 1 angegeben – mit Natriumhydroxid erhitzt und anschliessend mit ca. 80 g L-Milchsäure in Lösung gebracht. Nach der Verdünnung entstand 1,0 l einer klaren Flüssigkeit mit einem Germaniumgehalt von 7 mg/ml.

**Patentansprüche**

1. Komplexe aus Germanium mit Ascorbinsäure oder wasserlöslichen, physiologisch verträglichen organischen Carbonsäuren zur Verwendung als Arzneimittel, dadurch gekennzeichnet, dass man Germanium-IV-Oxid mit anorganischen Basen, vorzugsweise Natrium- oder Kaliumhydroxid, reagieren lässt und anschliessend mit einer der oben genannten Säuren oder Mischungen solcher Säuren, gegebenenfalls unter Zuhilfenahme von Mineralsäuren, umsetzt und das Reaktionsprodukt abtrennt.

2. Komplexe nach Anspruch 1 zur Verwendung als Zytostatika.

3. Komplexe nach Anspruch 1 oder 2, dadurch gekennzeichnet, dass die Säuren Asparginsäure, Glutaminsäure, Zitronensäure, Isozitronensäure, Apfelsäure, Bernsteinsäure, Ketoglutarsäure, Fumarsäure, Milchsäure und Ascorbinsäure sind.

4. Pharmazeutisches Präparat, dadurch gekennzeichnet, dass es als Wirkstoff einen Komplex nach einem der vorangehenden Ansprüche enthält.

**Revendications**

1. Complexes de germanium et d'acide ascorbique ou d'acides organiques solubles dans l'eau physiologiquement tolérables, pour utilisation comme médicaments, caractérisés en ce qu'on fait réagir de l'oxyde de germanium (IV) sur des bases inorganiques, de préférence l'hydroxyde de sodium ou l'hydroxyde de potassium, puis qu'on le fait réagir sur l'un des acides ou mélanges d'acides mentionnés ci-dessus, éventuellement en faisant appel à des acides minéraux, et qu'on sépare le produit de réaction.

2. Complexes selon la revendication 1, pour utilisation en tant que cytostatiques.

3. Complexes selon la revendication 1 ou 2, caractérisés en ce que les acides sont l'acide aspargique, l'acide glutamique, l'acide citrique, l'acide isocitrique, l'acide malique, l'acide succinique, l'acide cétoglutarique, l'acide fumarique, l'acide lactique et l'acide ascorbique.

4. Préparation pharmaceutique, caractérisée en ce qu'elle contient en tant que principe actif un complexe selon l'une des revendications précédentes.

## Claims

1. Complexes of germanium with ascorbic acid or water-soluble, physiologically compatible organic carbonic acids for use as medicament, characterized in that germanium-IV oxide is caused to react with inorganic bases, preferably sodium hydroxide or potassium hydroxide, and thereafter is reacted with one of the aforementioned acids or mixtures of such acids, possibly with the assistance of mineral acids, and the reaction product is separated.

2. Complexes according to claim 1, for use as cytostatic.

3. Complexes according to claim 1 or 2, characterized in that the acids are aspartic acid, glutamic acid, citric acid, isocitric acid, malic acid, succinic acid, ketoglutaric acid, fumaric acid, lactic acid and ascorbic acid.

4. Pharmaceutical preparation, characterized in that it contains, as active substance, a complex according to one of the preceding claims.